# EUROPÄISCHE PATENTSCHRIFT

(11) **EP 0 888 796 B1**
(45) Veröffentlichungstag und Bekanntmachung des Hinweises auf die Patenterteilung: **04.06.2003**
(21) Anmeldenummer: 98103373.1
(22) Anmeldetag: 26.02.1998
(51) Int. Cl.: A62B 9/02

(54) **Ventil**
Valve
Soupape

(30) Priorität: 04.04.1997 SE 9701240
(43) Veröffentlichungstag der Anmeldung: 07.01.1999
(73) Patentinhaber: Siemens-Elema AB, 171 95 Solna (SE)
(72) Erfinder: Arvidsson, Carl-Erik, 171 39 Solna (SE)

(56) Entgegenhaltungen:
- EP-A- 0 354 123
- DD-A- 273 982
- US-A- 5 265 594

## Beschreibung

Die Erfindung betrifft ein Ventil zum Regeln eines Mediumflußes, umfassend ein Ventilgehäuse mit einem Einlaß für das Medium, das geregelt werden soll, und einem Auslaß für einen geregelten Mediumfluß, einen Ventilsitz mit einer Ventilöffnung, ein bewegbares Verschlußteil, das so angeordnet ist, daß es von dem Mediumfluß in Öffnungsrichtung des Ventils beeinflußt wird und das die Ventilöffnung schließt und öffnet sowie den Mediumfluß durch die Ventilöffnung regelt, und Mittel zum Steuern der Lage des Verschlußteils, das zwischen dem Einlaß und der Seite des beweglichen Verschlußteils, die vom Ventilsitz abgewandt ist ein Mediumflußkanal angeordnet ist. Ein Ventil dieser Art ist durch die EP-A-354 123 bekannt. Das in dieser Schrift beschriebene Ventil ist im Aufbau sehr aufwendig und daher teuer.

Ein weiteres Ventil zum Regeln eines Mediumflußes ist durch die US-PS 5 265 594 bekannt. Es ist, insbesondere in Verbindung mit der Zufuhr von Gas zu einem Patienten, der an einem Beatmungsgerät angeschlossen ist, von großer Bedeutung, daß die Gasmenge mit Hilfe eines solchen Ventils mit großer Genauigkeit geregelt wird. Es ist auch sehr wichtig, daß das Verschlußteil, das hier aus einem Solenoid und einer Membran besteht, die Ventilöffnung hermetisch abdichtet, wenn die Stromzufuhr zum Solenoid ausgeschaltet wird. Dies ist in Verbindung mit dem in der US-Schrift beschriebenen Ventil sichergestellt, indem die Welle des Solenoids, die die Membran beeinflußt und auf die beim Ausschalten des Stroms keine Magnetkraft mehr wirkt, durch eine verhältnismäßig kräftige Feder beeinflußt wird, die Membran gegen den Ventilsitz zu drücken. Eine solche kräftige Feder kann beim Regeln des Gasflusses die Bewegungen des Solenoids hemmen. Ein verhältnismäßig kräftiges Solenoid kann selbstverständlich dieses Problem lösen. Ein solches Solenoid ist aber verhältnismäßig groß, ist daher platzraubend und hat einen hohen Stromverbrauch.

Der Erfindung liegt die Aufgabe zugrunde, ein Ventil der eingangs genannten Art zu schaffen, bei dem ein Gasfluß mit großer Genauigkeit geregelt werden und in einer verschlossenen Lage mittels einfacher und dadurch billiger Mittel hermetisch dicht schließen kann.

Diese Aufgabe ist erfindungsgemäß dadurch gelöst, daß das Verschlußteil eine Membran, die am Ventilgehäuse festgespannt ist und ein Solenoid mit einer Welle umfaßt, bei der die eine Endseite der Welle so angeordnet ist, daß sie die Lage der Membran beeinflußt, und bei der die andere Seite der Welle die Endseite darstellt. Wenn die Ventilöffnung mit Hilfe des Verschlußteils auf beschriebene Weise schließt, drückt das Gas von dem Einlaß, das in den erfindungsgemäßen Kanal hineingeströmt ist, gegen die erwähnte Seite des Verschlußteils und trägt dadurch dazu bei, die Ventilöffnung verschlossen zu halten. Hierdurch kann ein verhältnismäßig kleines Solenoid zum Öffnen und Verschließen der Ventilöffnung sowie zum Regeln des Gases durch die Ventilöffnung verwendet werden. Die Feder, die in Verbindung mit dem Stand der Technik erwähnt wurde, braucht hier lediglich so bemessen zu sein, daß sie das Gewicht der Solenoidwelle kompensiert.

In einer vorteilhaften Weiterentwicklung des Ventils nach der Erfindung wird vorgeschlagen, daß die Seite eine größere Flächengröße als die der Ventilöffnung aufweist. Hierdurch ist weiterhin sichergestellt, daß das Verschlußteil in einer Verschlußlage gegen den Ventilsitz hermetisch abdichtet.

In der DD 273 982 A1 ist ein Ventil zum Regeln eines Gasflusses beschrieben. Das Ventil ist mit einer Membran versehen, die die Ventilöffnung mit Hilfe eines Gases öffnet bzw. verschließt, indem das Gas mehr oder weniger gegen die Seite des Membrans, die dem Ventilsitz abgewandet ist, drückt. Der Gasdruck gegen die Membran ist verhältnismäßig groß, wobei die Membran nur dann vom Ventilsitz abheben kann, wenn der Gasdruck auf die Membran abnimmt. Daher ist die Membran mit einem durchgehenden Loch versehen, das dazu dient, einen Teil des Gases, das gegen die Membran drückt, herauszulassen und in den Raum der Ventilöffnung hineinzuleiten. Das Loch in der Membran wird mit Hilfe eines Solenoids geöffnet bzw. verschlossen. Das Solenoid steuert demnach nicht die Bewegungen der Membran, sondern ist lediglich dazu vorgesehen, den erwähnten Druckausgleich zustandezubringen. Dadurch, das Gas teils durch das Loch in der Membran und teils dadurch, daß die Membran vom Ventilsitz abhebt, in die Ventilöffnung hineinströmt, ist keine große Genauigkeit beim Regeln des Gasflusses durch das Ventil gegeben.

Die Erfindung ist nachfolgend anhand von zwei in den Zeichnungen dargestellten Ausführungsbeispielen näher erläutert. Es zeigen:

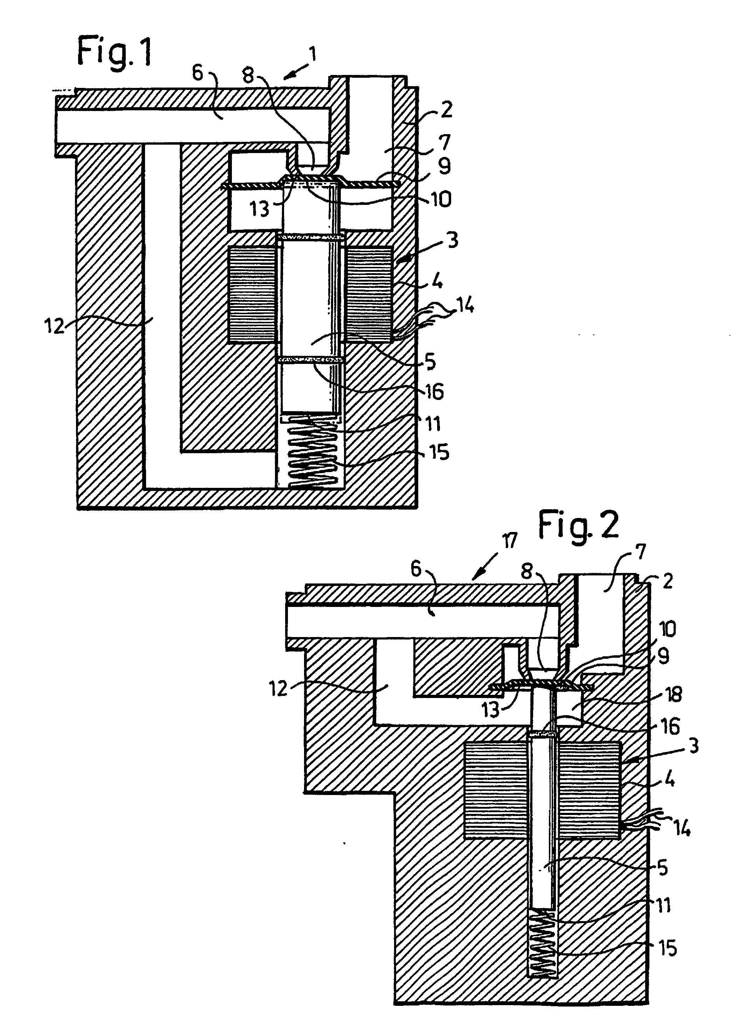

- FIG 1 und 2: Prinzipzeichnungen über Ventile nach der Erfindung in unterschiedlichen Ausführungsformen.

In der FIG 1 ist, teils im Schnitt, eine möglich Ausführungsform eines Ventils nach der Erfindung dargestellt. Das Ventil weist ein Ventilgehäuse 2 und ein Solenoid 3 auf, umfassend ein Solenoidgehäuse 4 und eine Welle 5, wobei das Solenoid 3 mit dem Ventilgehäuse 2 fest verbunden ist. Das Ventilgehäuse 2 ist mit einem durchgehenden Kanal für einen Mediumfluß, der geregelt werden soll, versehen, wobei der Kanal in einen Einlaß 6, einen Auslaß 7 und in eine dazwischen angeordnete Ventilöffnung 8 aufgeteilt ist. Genau vor der Ventilöffnung 8 ist eine Membran 9 angebracht, die am Ventilgehäuse 2 festgespannt ist und die dazu dient, mit Hilfe der einen Endseite 10 der Solenoidwelle 5, die Lage der Membran 9 und damit auch den Mediumfluß, z.B. ein Gas, zu einem Patienten in Verbindung mit einem hier nicht gezeigten Beatmungsgerät zu regeln. Zwischen dem erwähnten Einlaß 6 und der zweiten Endseite 11 der Solenoidwelle 5 ist in diesem Ausführungsbeispiel ein mit Gas vom Einlaß 6 gefüllter Mediumflußkanal 12 angeordnet. In der FIG ist gezeigt, das die Endseite 10 der Welle 5 des Solenoids 3 in einer verschlossenen Ventillage gegen die Membran 9 anliegt, die dadurch gegen den Ventilsitz 13 der Ventilöffnung 8 drückt. Um eine offene Ventillage, die hier mit strichpunktierten Konturen der Membran 9 und der Welle 5 gezeigt wird, zu erhalten, wird dem Solenoid 3 mehr oder weniger Strom über Leitungen 14 zugeführt, was die Welle 5 derart beeinflußt, daß sie in das Solenoidgehäuse 4 hineingezogen wird, d.h. nach unten in dieser FIG, wobei die Membran 9 durch die Federkraft des Materials und durch den Gasdruck auf der Einlaßseite auf gewünschte Weise von dem Ventilsitz 13 abhebt, so daß Gas über die Ventilöffnung 8 und über den Auslaß 7 weiter zum Patienten strömen kann. Wenn die Stromzufuhr zum Solenoid 3 ausgeschaltet wird, wirkt keine Magnetkraft mehr auf dessen Welle 5, wodurch die Welle 5 mit Hilfe einer Druckfeder 15 die Membran 9 gegen den Ventilsitz 13 derart drückt, daß die Ventilöffnung verschlossen wird. In dieser verschlossenen Lage der Ventilöffnung 8 drückt das Gas vom Mediumflußkanal 12 gegen die Endseite 11 der Solenoidwelle 5 und trägt dazu bei, die Welle 5 und die Membran 9 gegen den Ventilsitz 13 zu drücken. Durch Bemessen der Flächengröße des Mediumflußkanals 12 an der Endseite 11 der Welle 5, so daß sie größer als die Flächengröße der Ventilöffnung 8 ist, wird eine außerordentliche gute Dichtung erhalten. Die Welle 5 ist mit mindestens einer Dichtung 16 versehen, die verhindert, daß Gas in das Solenoid 3 eindringt, was das Solenoid 3 sonst zerstören könnte.

In der FIG 2 ist eine weitere Ausführungsform eines Ventils 17 nach der Erfindung gezeigt, das eine große Ähnlichkeit mit dem Ventil 1 nach der FIG 1 hat und das in Verbindung mit dieser FIG 1 beschrieben worden ist. Der Unterschied ist, daß sich der Mediumflußkanal 12 in diesem Beispiel vom Einlaß 6 zu einem Raum 18 im Anschluß zur Membran 9 erstreckt. In diesem Ausführungsbeispiel ist auch die Endseite 10 der Solenoidwelle 5 wie ein Flansch ausgebildet. Wenn die Welle 5 mit Hilfe der Druckfeder 15 die Membran 9 gegen den Ventilsitz 13 drückt, so daß die Ventilöffnung 8 verschlossen wird, drückt das Gas vom Mediumflußkanal 12 direkt gegen die flanschförmige Endseite der Welle 5 und gegen die freien Flächen der Membran 9.

Die Flächengröße der erwähnten Teile, gegen die das Gas im Raum 18 drückt, ist größer als die Flächengröße der Ventilöffnung 8. Dies bedeutet, daß eine extrem gute Abdichtung der Ventilöffnung 8 gegeben ist.

In den beiden Ausführungsbeispielen ist lediglich eine Feder erforderlich, die so bemessen ist, daß sie das Gewicht der Solenoidwelle 5 kompensiert. Durch diese verhältnismäßig weiche Feder ist lediglich ein verhältnismäßig schwaches, kleines, billiges und stromsparendes Solenoid zum Regeln des Gasflusses erforderlich.

Durch die Erfindung wird mittels einfacher und dadurch billiger Mittel ein in diesem Zusammenhang angestrebter Sicherheitsaspekt erzielt, nämlich, daß bei einem Stromausfall das Ventil hermetisch verschlossen und eine unkontrollierte Gasabgabe an den Patienten verhindert wird.

## Patentansprüche

1. Ventil (1) zum Regeln eines Mediumflußes, umfassend ein Ventilgehäuse (2) mit einem Einlaß (6) für das Medium, das geregelt werden soll, und einem Auslaß (7) für einen geregelten Mediumfluß, einen Ventilsitz (13) mit einer Ventilöffnung (8), ein bewegbares Verschlußteil (3, 9), das so angeordnet ist, daß es von dem Mediumfluß in Öffnungsrichtung des Ventils (1) beeinflußt wird und das die Ventilöffnung (8) schließt und öffnet sowie den Mediumfluß durch die Ventilöffnung (8) regelt, und Mittel zum Steuern der Lage des Verschlußteils (3, 9), daß zwischen dem Einlaß (6) und der Seite (10, 11) des beweglichen Verschlußteils (3, 9), die vom Ventilsitz (13) abgewandt ist, ein Mediumflußkanal (12) angeordnet ist, **dadurch gekennzeichnet, daß** das Verschlußteil (3, 9) eine Membran (9), die am Ventilgehäuse (2) festgespannt ist und ein Solenoid (3) mit einer Welle (5) umfaßt, bei der die eine Endseite (10) der Welle (5) so angeordnet ist, daß sie die Lage der Membran (9) beeinflußt und bei der die andere Seite der Welle (5) die Endseite (11) darstellt.

2. Ventil nach Anspruch 1, **dadurch gekennzeichnet, daß** die Seite (10, 11) eine größere Flächengröße als die der Ventilöffnung (8) aufweist.

## Claims

1. Valve (1) for regulating a medium flow, comprising a valve housing (2) with an inlet (6) for the medium which is to be regulated, and with an outlet (7) for a regulated medium flow, a valve seat (13) with a valve orifice (8), a movable closing part (3, 9) which is arranged in such a way that it is influenced by the medium flow in the opening direction of the valve (1) and which closes and opens the valve orifice (8) and regulates the medium flow through the valve orifice (8), and means for controlling the position of the closing part (3, 9), a medium-flow duct (12) being arranged between the inlet (6) and that face (10, 11) of the movable closing part (3, 9) which faces away from the valve seat (13), **characterized in that** the closing part (3, 9) comprises a diaphragm (9), which is firmly clamped to the valve housing (2), and a solenoid (3) with a shaft (5), in which one end face (10) of the shaft (5) is arranged in such a way that it influences the position of the diaphragm (9) and in which the other face of the shaft (5) forms the end face (11).

2. Valve according to Claim 1, **characterized in that** the face (10, 11) has a larger area size than that of the valve orifice (8).

## Revendications

1. Soupape (1) de réglage d'un débit de fluide, comprenant un corps (2) de soupape ayant une entrée (6) pour le fluide qui doit être réglé et une sortie (7) pour un débit de fluide réglé, un siège (13) de soupape ayant une ouverture (8) de soupape, un obturateur (3, 9) mobile qui est monté de façon à être influencé par le débit de fluide dans la direction d'ouverture de la soupape (1) et qui ferme et ouvre l'ouverture (8) de la soupape ainsi que règle le débit de fluide dans l'ouverture (8) de la soupape et des moyens de commande de la position de l'obturateur (3, 9), dans laquelle il est interposé, entre l'entrée (6) et la face (10, 11 ) de l'obturateur (3, 9) mobile qui est éloignée du siège (13) de la soupape, un canal (12) pour le débit de fluide, **caractérisée en ce que** l'obturateur (3, 9) comprend une membrane (9) qui est fixée sur le corps (2) de la soupape et un solénoïde (3) ayant un arbre (5), dans lequel la face (10) d'extrémité de l'arbre (5) est disposée de façon à influencer la position de la membrane (9) et dans lequel l'autre face de l'arbre (5) représente la face (11) d'extrémité.

2. Soupape selon la revendication 1 **caractérisée en ce que** la face (10, 11) a une surface plus grande que celle de l'ouverture (8) de la soupape.
